# EUROPEAN PATENT SPECIFICATION

(11) **EP 0 768 325 B1**
(45) Date of publication and mention of the grant of the patent: **30.05.2001**
(21) Application number: 96202872.6
(22) Date of filing: 15.10.1996
(51) Int. Cl.: C08G 18/08

(54) **Polyol blends**
Polyolgemische
Mélanges de polyols

(30) Priority: 16.10.1995 EP 95307467
(43) Date of publication of application: 16.04.1997
(73) Proprietor: SHELL INTERNATIONALE RESEARCH MAATSCHAPPIJ B.V., 2596 HR Den Haag (NL)
(72) Inventor: Bleijenberg, Karel Cornelis, 1330 Rixensart (BE); Broennum, Thomas, 1348 Ottignies (BE); Fleurent, Hilde, 3000 Leuven (BE); Sangha, Parminder Singh, 1031 CM Amsterdam (NL)

(56) References cited:
- EP-A- 0 097 458
- US-A- 4 569 952
- WOODS: "THE ICI POLYURETHANES BOOK" 1988 , WILEY AND SONS , GB XP002022792 147280 * page 14, line 1 - line 13 * * page 17, line 42 - page 18, line 12 *
- OERTEL: "KUNSTSTOFF HANDBUCH: POLYURETHANE" 1984 , HANSER VERLAG , MÜNCHEN,DE XP002022793 * page 136, paragraph 4.3.2 - page 137 *

## Description

The invention is concerned with a method of preparing a blend of polyols. Polyols, in particular polyether polyols or polyester polyols, are used in the manufacture of polyurethane articles. Usually a polyol producer manufactures a substantial number of different polyols having a range of characteristics such as functionality, hydroxyl value, nitrogen content, aromaticity and viscosity. This is particularly the case for so-called rigid polyols which are used in the manufacture of rigid polyurethane foam where the wide variety of applications requires an extensive range of foam properties and hence a significant number of different polyol grades. Consequently the polyol producer has to invest in a large number of storage vessels. The manufacturing operation can be simplified by producing a limited number of base polyol grades from which a larger number of commercial grades can be prepared by blending. However, this does not in itself overcome the storage problem in view of the need to store those blended polyol grades in addition to the base polyol grades.

The Applicants have now found that this problem may be overcome by preparing a polyol blend ready for transportation, which comprises withdrawing from their respective storage tanks a plurality of streams of different base polyols, continuously and simultaneously feeding said streams of base polyols in a predetermined ratio into a blender, and continuously discharging the resulting polyol blend into a transporter tank. This blending process thus makes it possible to produce from a small number of properly selected base polyols an extensive variety of (blended) polyols which supply all the different customer requirements, not only in respect of OH values and functionality, but also matching the criteria for their polyurethane process and polyurethane end product.

Although continuous mixing/discharge of various liquids is known (see e.g. EP 0 097 458 A), this technique has not been applied hitherto by polyol manufacturers. The main advantage of the invention is that the polyol manufacturer has to invest in storage facilities only for the limited number of base polyol grades, plus in-line mixing equipment - which is smaller and easier to clean than batch mixing equipment. Moreover, the utilisation of in-line blending makes it economically practicable to produce any desired quantity of a specific blend; if the blend were produced by batch mixing, it would be technically difficult to produce very small quantities (effectively "tailor-made" product grades), whilst large quantities would require either large volume mixers or production via several, successive batch blendings (requiring the storage of successive batches). Also, the inherent flexibility of the blending concept makes it possible to provide new polyol grades simply by modifying the selection of base polyol and/or their blending ratios.

The mixing system used to blend the base polyols should be 'in-line' (i.e. continuous rather than batch), and comprises multiple feed lines to one or more mixing areas and a discharge nozzle. In its simplest form it can be simply a mixing space within a pipe.

The process may be carried out in conventional equipment utilised for blending different feedstreams, with the addition of control means and switching mechanism by which it is possible to shut down a feedstream and switch to another feedstream when a different polyol grade is to be prepared, or to change the relative feed ratios of the base polyols utilised from the predetermined rate required for one polyol grade to a different predetermined rate as required for a different polyol grade.

Clearly, the choice of the individual, different base polyols is an important factor in the ability to produce the desired range of properties through blending of different ratios/components. In general, it has been found that several different base polyols are usually required; naturally, it is technically simpler and economically preferable to operate with the minimum necessary range of base polyols, and it has been found that no more than 6, often only 3 or 4, are usually sufficient to produce the entire range of commercial polyol grades.

The different base polyols are suitably selected to encompass a wide range of hydroxyl values and functionalities. Preferably the base polyols encompass hydroxyl values in the range of from 200 to 700; functionalities in the range of from 2.0 to 5.5; and include at least one aromatic polyol. Suitably the individual different base polyols comprise:- a) at least two with functionality below 3.5, one having an OH value above 500 and another having an OH value less than 300; b) at least one having a functionality of at least 4.5 and OH value in the range of from 200 to 600; c) at least one having an aromatic content of at least 15%; and d) at least one having a nitrogen content of at least 2.5% m/m. Naturally, it may be possible to combine requirements c) and/or d) with a) and/or b) in a single base polymer.

Additional flexibility in the final blended polyol can be obtained by feeding into the blender an additional stream of a diol or triol, such as glycerol or trimethylol propane.

The following examples serve to illustrate the types of base polyol that can serve the needs of the present process, and the range of polyol blends corresponding to commercial grades that can be generated by appropriate blending of those base polyols.

| Set of base polyols | | | |
|---|---|---|---|
| Base Polyol | Initiator/oxide | OH-value (mg KOH/g) | Functionality (eq/mole) |
| BP-A | Glycerol/PO | 250 | 3.0 |
| BP-B | Glycerol/PO | 570 | 3.0 |
| BP-C | Sorbitol/H₂O//PO | 475 | 5.3 |
| BP-D ∗ | EDA/PO | 630 | 4.0 |
| (PO = propylene oxide; EDA = ethylene diamine); | | | |

| | | | |
|---|---|---|---|
| ∗ having a nitrogen content of at least 2.5% m/m. | | | |

| Commercial polyol grades whose properties can be matched by blend of base polyols | | | | | |
|---|---|---|---|---|---|
| Commercial grade equivalent | Ratio of Base polyols (% by volume) | | | | |
| | BP-A | BP-B | BP-C | BP-D | Glycerol |
| CG-1 | - | 17.7 | 43.2 | 36.3 | 2.8 |
| CG-2 | 13.3 | 29.2 | 19.8 | 37.8 | - |
| CG-3 | - | 21.9 | 76.3 | - | 1.8 |
| CG-4 | 11.0 | 9.0 | 70.7 | 9.4 | - |
| CG-5 | 9.8 | 27.9 | 60.6 | - | 1.7 |

Each of these CG Blends was evaluated in standard foam formulations and foaming conditions appropriate to the end application (eg. lamination or pipe cladding), and the quality of resulting foams matched those from the corresponding commercial grade.

## Claims

1. Method of preparing a polyol blend ready for transportation, which comprises withdrawing from their respective storage tanks a plurality of streams of different base polyols, continuously and simultaneously feeding said streams of base polyols in a predetermined ratio into a blender, and continuously discharging the resulting polyol blend into a transporter tank.

2. Method as claimed in claim 1, wherein the base polyols fed to the blender are selected from up to 6 different base polyols.

3. Method as claimed in claim 1 or claim 2, wherein the different base polyols are selected to encompass a wide range of hydroxy values and functionalities.

4. Method as claimed in claim 3, wherein the different base polyols encompass hydroxyl values in the range of from 200 to 700; functionalities in the range of from 2.0 to 5.5; and include at least one aromatic polyol.

5. Method as claimed in claim 4, wherein the different base polyols comprise:- at least two with functionality below 3.5, one having an OH value above 500 and another having an OH value less than 300; at least one having a functionality of at least 4.5 and an OH value in the range of from 200 to 600; at least one having an aromatic content of at least 15%; and at least one having a nitrogen content of at least 2.5% m/m.

6. Method as claimed in any one of claims 1 to 5, wherein a stream of aliphatic diol or triol is fed into the blender simultaneously with the streams of base polyols.

7. Method as claimed in claim 6, wherein the aliphatic diol or triol is glycerol.

## Patentansprüche

1. Verfahren zur Herstellung eines transportfertigen Polyolgemisches, das ein Entnehmen mehrerer Ströme unterschiedlicher Grundpolyole aus ihren jeweiligen Vorratstanks, ein kontinuierliches und gleichzeitiges Einspeisen dieser Grundpolyolströme in einem vorbestimmten Verhältnis in einen Mischer und ein kontinuierliches Entleeren des gebildeten Polyolgemisches in einen Transporttank umfaßt.

2. Verfahren nach Anspruch 1, worin die dem Mischer zugeführten Grundpolyole unter bis zu sechs verschiedenen Grundpolyolen ausgewählt werden.

3. Verfahren nach Anspruch 1 oder Anspruch 2, worin die unterschiedlichen Grundpolyole so ausgewählt werden, daß ein weiter Bereich von Hydroxylzahlen und Funktionalitäten umfaßt wird.

4. Verfahren nach Anspruch 3, worin die unterschiedlichen Grundpolyole Hydroxylzahlen im Bereich von 200 bis 700 und Funktionalitäten im Bereich von 2,0 bis 5,5 umfassen und wenigstens ein aromatisches Polyol einschließen.

5. Verfahren nach Anspruch 4, worin die unterschiedlichen Grundpolyole umfassen: wenigstens zwei mit Funktionalitäten unter 3,5, von denen eines eine OH-Zahl von über 500 und ein weiteres eine OH-Zahl von unter 300 aufweisen; wenigstens eines eine Funktionalität von wenigstens 4,5 und eine OH-Zahl im Bereich von 200 bis 600 aufweist; wenigstens eines einen Aromatengehalt von wenigstens 15% hat; und wenigstens eines einen Stickstoffgehalt von wenigstens 2,5% Masse/Masse aufweist.

6. Verfahren nach einem der Ansprüche 1 bis 5, worin ein Strom aus aliphatischem Diol oder Triol gleichzeitig mit den Strömen aus Grundpolyolen in den Mischer eingespeist wird.

7. Verfahren nach Anspruch 6, worin das aliphatische Diol oder Triol Glycerin ist.

## Revendications

1. Procédé de préparation d'un mélange de polyols prêt au transport, qui comprend le prélèvement de leurs cuves de stockage respectives d'une pluralité de courants de différents polyols de base, l'apport continu et simultané desdits courants de polyols de base dans un rapport prédéterminé dans un mélangeur et le déchargement continu du mélange de polyols résultant dans une cuve de transporteur.

2. Procédé suivant la revendication 1, dans lequel les polyols de base amenés au mélangeur sont choisis parmi jusqu'à 6 polyols de base différents.

3. Procédé suivant l'une ou l'autre des revendications 1 et 2, dans lequel les différents polyols de base sont choisis pour couvrir une gamme étendue d'indices d'hydroxyle et de fonctionnalités.

4. Procédé suivant la revendication 3, dans lequel les différents polyols de base recouvrent des indices d'hydroxyle dans la gamme de 200 à 700, des fonctionnalités dans la gamme de 2,0 à 5,5, et comprennent au moins un polyol aromatique.

5. Procédé suivant la revendication 4, dans lequel les différents polyols de base comprennent : - au moins deux avec une fonctionnalité en dessous de 3,5, un ayant un indice d'OH au-dessus de 500 et un autre ayant un indice d'OH inférieur à 300; au moins un ayant une fonctionnalité d'au moins 4,5 et un indice d'OH allant de 200 à 600; au moins un ayant une teneur en aromatique d'au moins 15%; et au moins un ayant une teneur en azote d'au moins 2,5% m/m.

6. Procédé suivant l'une quelconque des revendications 1 à 5, dans lequel un courant de diol ou triol aliphatique est amené dans le mélangeur simultanément aux courants de polyols de base.

7. Procédé suivant la revendication 6, dans lequel le diol ou triol aliphatique est du glycérol.
